# EUROPÄISCHE PATENTSCHRIFT

(11) **EP 3 977 959 B1**
(45) Veröffentlichungstag und Bekanntmachung des Hinweises auf die Patenterteilung: **31.07.2024**
(21) Anmeldenummer: 20199437.3
(22) Anmeldetag: 30.09.2020
(51) Int. Cl.: A61C 13/00, C04B 35/48

(54) **VERFAHREN ZUR HERSTELLUNG EINES DENTALEN FORMKÖRPERS**
METHOD FOR PRODUCTION OF A DENTAL MOULD
PROCÉDÉ DE FABRICATION D'UN CORPS MOULÉ DENTAIRE

(43) Veröffentlichungstag der Anmeldung: 06.04.2022
(73) Patentinhaber: Ivoclar Vivadent AG, 9494 Schaan (LI)
(72) Erfinder: RAMPF, Markus, 7212 Seewis Dorf (CH); MATHIS, Nina Maria, 9491 Ruggel (LI); BONDERER, Lorenz Josef, 7320 Sargans (CH); RITZBERGER, Christian, 9472 Grabs (CH)
(74) Vertreter: Uexküll & Stolberg

(56) Entgegenhaltungen:
- EP-A1- 3 659 547
- EP-A1- 3 659 574
- EP-A1- 3 659 989
- EP-A1- 3 663 272
- WO-A1-2020/138163
- US-A1- 2017 152 193

## Beschreibung

Die Erfindung betrifft ein Verfahren zur Herstellung eines dentalen Formkörpers und insbesondere zur Herstellung von dentalen Rohlingen oder dentalen Restaurationen.

Oxidkeramiken, wie z.B. Zirkonoxidkeramiken, finden wegen ihrer vorteilhaften mechanischen Eigenschaften zur Herstellung von dentalen Restaurationen verbreitet Anwendung. Insbesondere Zirkonoxidmaterialien, die auf mit Y₂O₃ stabilisierten tetragonalen Polykristallen basieren, sind wegen ihrer vorteilhaften mechanischen Eigenschaften als Ausgangsmaterial für dentale Restaurationen geeignet.

Zirkonoxidkeramiken können mithilfe von unterschiedlichen Verfahren hergestellt werden. Bevorzugte Verfahren sind (i) axiales Pressen oder kaltisostatisches Pressen (CIP) gefolgt von konventionellem Sintern, (ii) Schlickergießen gefolgt von konventionellem Sintern und (iii) Heißpressen (HP), heißisostatisches Pressen (HIP) oder Spark-Plasma Sintern (SPS). Dabei umfasst die Herstellung von dentalen Restaurationen aus Zirkonoxid, wie auch in der WO 2018/115529 A1 beschrieben, typischerweise zwei thermische Verdichtungsschritte, die durch einen Formgebungsschritt getrennt sind. Die Zirkonoxid-Ausgangsmaterialien werden typischerweise zunächst gegossen oder unter Verwendung von Druck mechanisch verdichtet und dann zu einem intermediären offenporigen Zustand vorgesintert, um einen Rohling herzustellen. Dieser Rohling eignet sich für eine Formgebung oder Vorformgebung, z.B. durch maschinelle Bearbeitung in einem CAD/CAM-Verfahren. Der geformte Rohling kann dann in einem weiteren Sinterschritt einer abschließenden thermischen Verdichtung unterzogen werden.

Ein anderer Ansatz zur Herstellung von dentalen Zirkonoxid-Restaurationen verwendet aufbauende Fertigungsverfahren, wie z.B. das in der EP 3 659 547 A1 beschriebene 3D-Inkjet-Druckverfahren. Dabei wird üblicherweise ein dreidimensionaler Körper hergestellt, indem geeignete Suspensionen in einem Druckverfahren übereinander geschichtet und dann gesintert werden.

EP3663272 offenbart ein Verfahren zur Herstellung eines dentalen Formkörpers.

Bei der Herstellung dentaler Restaurationen ist es wünschenswert, die komplexe Farbgebung natürlicher Zähne mit Farbverläufen und 3D-Farbeffekten naturgetreu zu imitieren. Besonders für dentale Restaurationen aus Zirkonoxid stellt dies eine große Herausforderung dar, weshalb gerade im Bereich der Frontzähne andere Materialien, wie z.B. Lithiumsilikat-Glaskeramiken, trotz deren häufig dem Zirkonoxid unterlegenen mechanischen Eigenschaften bevorzugt werden. Zur Einfärbung von dentalen Restaurationen aus Zirkonoxid sind eine Reihe von Möglichkeiten bekannt.

Aufbauende Fertigungsverfahren erlauben durch die Verwendung unterschiedlich gefärbter Suspensionen in der Regel auch die Herstellung von dentalen Zirkonoxid-Restaurationen mit komplexer Farbgebung. Da solche aufbauenden Verfahren allerdings sehr aufwendig sind, werden in der Regel Verfahren mit einem maschinellen Formgebungsschritt bevorzugt.

Ein weiterer üblicher Ansatz zur Färbung von dentalen Zirkonoxid-Restaurationen verwendet die Infiltration des Keramikmaterials im porösen Zustand mit farbigen Metallverbindungen. Dabei wird in der Regel der zu färbende Körper getrocknet, ganz oder teilweise mit einer färbenden Lösung infiltriert, die Oberfläche gereinigt und getrocknet, gegebenenfalls das Verfahren mit weiteren Färbelösungen wiederholt und schließlich der gefärbte Körper gesintert.

In einem anderen Ansatz werden zunächst gefärbte Zirkonoxid-Pulver hergestellt, indem Zirkonoxid zusammen mit färbenden Elementen kopräzipitiert wird oder indem ein Zirkonoxid-Pulver mit Lösungen von färbenden Verbindungen in Kontakt gebracht wird. Solche gefärbten Zirkonoxid-Pulver können z.B. in Pulverform oder in Form von Suspensionen übereinander geschichtet werden, um mehrfarbige dentale Restaurationen herzustellen. Nachteilig ist dabei jedoch, dass es zu nicht kontrollierbaren Mischeffekten an der Grenzfläche der Schichten kommt. Außerdem können nur überwiegend ebenmäßige Schichtanordnungen erzielt werden, was die Erzeugung komplexer dreidimensionaler Farbgebungen verhindert, bei denen z.B. der Kern der Restauration eine andere Farbe als die um den Kern liegende Schale aufweist.

Die US 2015/0282905 A1 offenbart ein Verfahren zur Herstellung von dentalen Zirkonoxid-Rohlingen, bei dem Zirkonoxid-Suspensionen, die eine polymerisierbare Komponente wie z.B. Acrylat oder Methacrylat enthalten, mittels einer Wärme- oder Strahlungsbehandlung polymerisiert werden, wodurch Suspensionen mit erhöhter Viskosität entstehen. Es werden zwei oder mehr Zirkonoxid-Suspensionen mit Feststoffgehalten von lediglich bis zu 35 Gew.-% verwendet, die sich hinsichtlich der Zirkonoxid-Kristallphasen unterscheiden, weshalb der hergestellte Rohling Bereiche mit unterschiedlichen mechanischen Eigenschaften, insbesondere unterschiedlicher Härte, und unterschiedlicher Transluzenz aufweist. Zur Erzielung von Farbigkeit oder von Mehrfarbigkeit ist eine Behandlung der Rohlinge mit Färbelösungen erforderlich. Das Verfahren ist für die Verwendung kommerzieller Zirkonoxid-Pulver ungeeignet, weil dies zu Einschränkungen bei der Bearbeitbarkeit von Rohlingen und zu nachteiligen optischen Eigenschaften führt.

Der Erfindung liegt die Aufgabe zugrunde, ein Verfahren zur Herstellung von dentalen Formkörpern zur Verfügung zu stellen, das effizient ist, eine einfache Formgebung ermöglicht und auch für die Herstellung mehrfarbiger Formkörper geeignet ist. Das Verfahren sollte ferner mit einer geringen Schwindung einhergehen und insbesondere die Erzeugung komplexer Farbgebungen ermöglichen.

Diese Aufgabe wird überraschenderweise durch das Verfahren zur Herstellung eines dentalen Formkörpers gemäß den Ansprüchen 1 bis 16 gelöst. Die Erfindung betrifft außerdem den vorgesinterten dentalen Formkörper nach Anspruch 17 sowie die Verwendung einer

Suspension eines Zirkonoxid-Ausgangsmaterials nach Anspruch 18.

Das erfindungsgemäße Verfahren zur Herstellung eines dentalen Formkörpers zeichnet sich dadurch aus, dass mindestens zwei Suspensionen eines Zirkonoxid-Ausgangsmaterials mit unterschiedlichen Zusammensetzungen geliert werden, indem eine erste Suspension mindestens teilweise geliert wird und dann eine zweite Suspension auf die erste aufgetragen und ebenfalls geliert wird, wobei die Suspensionen ein Geliermittel enthalten und wobei die Suspensionen unterschiedliche Farben aufweisen.

Es ist überraschenderweise festgestellt worden, dass das erfindungsgemäße Verfahren eine einfache Fertigung von dentalen Formkörpern auch aus kommerziell erhältlichen Zirkonoxid-Pulvern ermöglicht. Es können überraschenderweise auch aus kommerziellen Zirkonoxid-Pulvern hergestellte Rohlinge einer einfachen maschinellen Bearbeitung unterzogen werden, und dentale Restaurationen, die aus kommerziell erhältlichen Zirkonoxid-Pulvern unter Verwendung des erfindungsgemäßen Verfahrens hergestellt wurden, weisen überraschenderweise vorteilhafte optische Eigenschaften auf.

Es hat sich ferner überraschend gezeigt, dass das Verfahren eine einfache Formgebung ermöglicht und die Herstellung von unterschiedlichen dentalen Formkörpern mit einer hohen Qualität der Kanten und Oberflächen ermöglicht. Das Verfahren ermöglicht zudem überraschenderweise eine einfache Handhabung der gelierten Suspension und die Herstellung von Formkörpern mit dünnen Wandstärken. Das Verfahren ermöglicht daher sogar die Herstellung dentaler Restaurationen in einem Gießverfahren, bei dem auf eine maschinelle Formgebung vollständig oder teilweise verzichtet werden kann.

Es ist zudem überraschenderweise festgestellt worden, dass das Verfahren auch Vorteile für die Herstellung von mehrfarbigen dentalen Formkörpern bietet, da es Mischeffekte, die typischerweise an der Grenzfläche von zwei Schichten auftreten, vermeidet. Das Verfahren ermöglicht ferner überraschenderweise die Herstellung von mehrfarbigen dentalen Formkörpern mit komplexen, auch dreidimensionalen Farbgebungen und vielfältigen Schichtanordnungen. Darüber hinaus wurde festgestellt, dass das Gefüge von dentalen Restaurationen mit mehreren unterschiedlichen Schichten sogar an den Grenzflächen eine überraschend hohe Belastbarkeit aufweist.

Die Begriffe "Farbe" und "gefärbt" beziehen sich im Sinne der Erfindung auf den Farbwert, die Helligkeit und/oder die Transluzenz, insbesondere auf den Farbwert und/oder die Helligkeit.

Farbwerte und Helligkeiten können durch den L*a*b-Wert, insbesondere nach DIN EN ISO 11664-4, oder nach einem in der Dentalindustrie gängigen Farbschlüssel bestimmt werden. Die Farbmessung kann mit handelsüblichen Messgeräten, wie einem Spektralphotometer CM-3700d (Konica Minolta) durchgeführt werden. Beispiele für Farbschlüssel sind der Vitapan classical^{®} und der Vita 3D Master^{®}, beide von der VITA Zahnfabrik H. Rauter GmbH & Co. KG, und der Chromascop^{®} der Ivoclar Vivadent AG.

Die Transluzenz ist die Lichtdurchlässigkeit eines Materials, d.h. das Verhältnis von durchgelassener zu einfallender Lichtintensität. Die Transluzenz kann in Form des Kontrastwertes (CR-Wert) gemäß dem British Standard 5612 bestimmt werden.

Zwei Suspensionen weisen im Sinne der vorliegenden Erfindung unterschiedliche Farben auf, wenn sie oder daraus hergestellte dichtgesinterte dentale Formkörper sich hinsichtlich der L*a*b-Werte, der mittels Farbschlüssel bestimmten Farbwerte und/oder der Transluzenz, insbesondere der L*a*b-Werte und/oder der Farbwerte, unterscheiden.

Die im erfindungsgemäßen Verfahren eingesetzte Suspension enthält ein Geliermittel. Im Sinne der vorliegenden Erfindung ist ein Geliermittel ein Mittel, durch das die Suspension in einen nicht mehr fließfähigen Zustand, vorzugsweise einen viskoelastischen Zustand, versetzt werden kann. "Viskoelastisch" sind im Sinne der vorliegenden Erfindung Suspensionen, die nicht fließfähig sind und zudem elastische Eigenschaften aufweisen. Das Überführen der Suspension in einen nicht fließfähigen, vorzugsweise viskoelastischen Zustand wird als "Gelieren" und das erhaltene Material als "Gel" oder "gelierte Suspension" bezeichnet.

In einer Ausführungsform des erfindungsgemäßen Verfahrens ist die gelierte Suspension viskoelastisch und vorzugsweise entformbar. Eine gelierte Suspension ist im Sinne der Erfindung "entformbar", wenn sie so aus einer Form entnommen werden kann, dass ihre äußere Gestalt nach dem Entnehmen im Wesentlichen ein Gegenstück zur verwendeten Form darstellt.

In einer bevorzugten Ausführungsform zeichnet sich die gelierte Suspension dadurch aus, dass in einem Nadel-Penetrationstest mit einem Penetrometer (z.B. PNR 10 von SUR Berlin) unter Verwendung eines Fallstabs mit einem Gewicht von 15 g und einer runden Edelstahl-Stiftnadel mit einem Gewicht von 3 g bei einer Belastungszeit von 0,2 s die Stiftnadel in die Suspension eindringt, jedoch nicht mehr als 20 mm, wobei die Stiftnadel einen Durchmesser von 3 mm, eine Gesamtlänge von 58 mm und eine gerade kegelförmige Spitze mit einer Höhe von 5,6 mm und einem Öffnungswinkel von 30° aufweist. Geeignet ist z.B. die Stiftnadel vom Typ 18-0222 der Petrotest GmbH. Dabei wird die Suspension unmittelbar vor der Durchführung des Test entweder zerteilt oder aus einer Form entnommen und dann der Zustand der Suspension in einem Bereich bestimmt, der sich davor im Inneren der Suspension oder in Kontakt mit der Form befand.

Das Zirkonoxid-Ausgangsmaterial liegt üblicherweise als Suspension in einem flüssigen Medium vor. Das flüssige Medium kann anorganische und/oder organische Lösungsmittel enthalten. Ein bevorzugtes anorganisches Lösungsmittel ist Wasser. Bevorzugte organische Lösungsmittel sind mit Wasser mischbare organische Lösungsmittel, insbesondere Alkohole, Ketone, Ester, Ether und Mischungen davon. Es können auch Mischungen von Wasser mit einem oder mehreren organischen Lösungsmitteln eingesetzt werden. Das flüssige Medium enthält insbesondere Wasser. Besonders bevorzugt besteht das flüssige Medium im Wesentlichen oder ausschließlich aus Wasser. Im flüssigen Medium können auch flüssige Zusatzstoffe enthalten sein.

Die feste Komponente der Suspension besteht aus dem Zirkonoxid-Ausgangsmaterial und gegebenenfalls festen Zusatzstoffen, wobei das Zirkonoxid-Ausgangsmaterial bevorzugt mindestens 80 Gew.-%, insbesondere mindestens 90 Gew.-% und besonders bevorzugt mindestens 95 Gew.-%, bezogen auf das Gewicht der festen Komponente, ausmacht.

In einer bevorzugten Ausführungsform enthält die Suspension 55 bis 70 Gew.-%, vorzugsweise 60 bis 70 Gew.-%, besonders bevorzugt 65 bis 70 Gew.-% Zirkonoxid-Ausgangsmaterial.

Es ist überraschenderweise festgestellt worden, dass die Verwendung von Suspensionen mit einem hohen Gehalt an Zirkonoxid die Herstellung von dentalen Formkörpern erlaubt, wobei die typischerweise beim Trocknen und Sintern auftretende Schwindung geringer als bei Verfahren ist, bei denen Suspensionen mit geringeren Feststoffgehalten verwendet werden. Ferner hat sich überraschend gezeigt, dass der hohe Gehalt an Zirkonoxid eine effizientere Herstellung von dentalen Formkörpern erlaubt, da der Gelierschritt und ein Trocknungsschritt verkürzt werden können.

Das Zirkonoxid-Ausgangsmaterial ist in der Regel ein partikuläres Zirkonoxid, vorzugsweise ein Zirkonoxid-Pulver. Das Zirkonoxid-Pulver besteht bevorzugt aus Zirkonoxid-Partikeln, die eine d₅₀-Partikelgröße, bezogen auf das Volumen des Pulvers, von 50 bis 250 nm, insbesondere 60 bis 250 nm, besonders bevorzugt 80 bis 250 nm, haben. Es ist zudem bevorzugt, dass das Zirkonoxid-Pulver eine Primärpartikelgröße von 30 bis 100 nm, bestimmt mittels dynamischer Lichtstreuung (DLS), aufweist.

Das Zirkonoxid-Ausgangsmaterial kann auf un- oder teilstabilisiertem monoklinen Zirkonoxid, teilstabilisiertem tetragonalen Zirkonoxid oder vollstabilisiertem kubischen Zirkonoxid oder deren Mischungen, insbesondere auf un- oder teilstabilisiertem monoklinen Zirkonoxid oder teilstabilisiertem tetragonalen Zirkonoxid oder deren Mischungen basieren. Bevorzugt ist das Zirkonoxid-Ausgangsmaterial ferner polykristallin und besonders bevorzugt im Wesentlichen polykristallines tetragonales Zirkonoxid (TZP).

In einer weiteren bevorzugten Ausführungsform enthält das Zirkonoxid-Ausgangsmaterial Y₂O₃, La₂O₃, CeO₂, MgO und/oder CaO, vorzugsweise in einer Menge von 2 bis 14 Mol-%, insbesondere 2 bis 8 Mol-%, besonders bevorzugt 3 bis 5 Mol-%, bezogen auf den Gehalt an Zirkonoxid. Besonders bevorzugt enthält das Zirkonoxid-Ausgangsmaterial Y₂O₃.

Das im erfindungsgemäßen Verfahren eingesetzte Zirkonoxid-Ausgangsmaterial ist gefärbt. Die gewünschte Einfärbung kann dadurch erzielt werden, dass das Zirkonoxid-Ausgangsmaterial mit einem oder mehreren färbenden Elementen kopräzipitiert wird. Diese Färbung wird auch als Dotierung bezeichnet und erfolgt üblicherweise während der Herstellung des Zirkonoxid-Ausgangsmaterials. Als färbende Elemente sind z.B. Fe, Mn, Cr, Ni, Ti, Co, Pr, Ce, Eu, Gd, Nd, V, Yb, Ce, Tb, Er und Bi geeignet. Besonders bevorzugt weist das Zirkonoxid-Ausgangsmaterial eine Farbe auf, die der Farbe des natürlichen Zahnmaterials entspricht.

Die im erfindungsgemäßen Verfahren verwendete Suspension enthält ferner ein Geliermittel. Das Geliermittel kann in der Suspension in der festen Komponente und/oder im flüssigen Medium enthalten sein. Es kann zudem von den Umgebungsbedingungen, insbesondere von der Temperatur, abhängen, ob das Geliermittel in festem oder flüssigem Zustand vorliegt.

Vorzugsweise kann das Gelieren der Suspension durch den Einfluss von einem oder mehreren Parametern, die im Sinne der Erfindung als Gelierparameter bezeichnet werden, ausgelöst und/oder gesteuert werden. Geeignete Gelierparameter sind eine Änderung von Temperatur oder pH-Wert der Suspension, eine Inkubationszeit und eine Bestrahlung. Vorzugsweise kann das Gelieren durch eine Temperaturänderung der Suspension ausgelöst und/oder gesteuert werden.

In einer besonders bevorzugten Ausführungsform des erfindungsgemäßen Verfahrens wird die Suspension durch Abkühlen geliert. Die Temperatur vor dem Abkühlen liegt vorzugsweise im Bereich von 50°C bis 100°C, insbesondere 60°C bis 80°C. Die Temperatur nach dem Abkühlen liegt vorzugsweise im Bereich von -10°C bis 40°C, vorzugsweise 0°C bis 25°C.

Es ist bevorzugt, dass das im erfindungsgemäßen Verfahren eingesetzte Geliermittel ein Polysaccharid, ein Protein oder eine Mischung davon, vorzugsweise Agarose, Agar, Alginat, Gellan, Carrageen, Pektin, Chitosan, Chitin, Gelatine oder eine Mischung davon, und besonders bevorzugt Agarose, ist.

Es können auch Geliermittel verwendet werden, die nicht natürlichen Ursprungs sind, wie z.B. ein Isobutyl/Maleinsäureanhydrid-Copolymer.

Die Suspension enthält vorzugsweise mindestens 0,01 Gew.-%, insbesondere 0,01 bis 1,5 Gew.-%, weiter bevorzugt 0,02 bis 1 Gew.-% und besonders bevorzugt 0,03 bis 0,5 Gew.-% Geliermittel.

In einer bevorzugten Ausführungsform enthält die Suspension mindestens 0,03 Gew.-%, insbesondere 0,03 bis 0,5 Gew.-% und besonders bevorzugt 0,03 bis 0,4 Gew.-% Agarose.

In einer weiteren bevorzugten Ausführungsform enthält die Suspension mindestens 0,1 Gew.-%, insbesondere 0,1 bis 1 Gew.-% und besonders bevorzugt 0,1 bis 0,8 Gew.-% Agarose, bezogen auf das Gewicht des flüssigen Mediums.

Die Suspension kann zusätzlich zu dem Zirkonoxid-Ausgangsmaterial, dem Geliermittel und dem flüssigen Medium auch feste und/oder flüssige Zusatzstoffe enthalten. Es können in der Suspension beispielsweise Dispergiermittel, Bindemittel, Mittel zur Einstellung des pH-Werts, Stabilisierungsmittel und/oder Entschäumer, wie sie in der EP 3 659 547 A1 beschriebenen sind, enthalten sein.

In einer bevorzugten Ausführungsform enthält die Suspension Entschäumer zur Vermeidung von Luftblasen. Der oder die Entschäumer werden typischerweise in einer Menge von 0,001 bis 1 Gew.-%, vorzugsweise 0,001 bis 0,5 Gew.-% und besonders bevorzugt 0,001 bis 0,1 Gew.-%, bezogen auf die Menge an Feststoff in der Suspension, in dem flüssigen Medium verwendet. Beispiele für geeignete Entschäumer sind Paraffin, Silikonöle, Alkylpolysiloxane, höhere Alkohole, Propylenglykol, Ethylenoxid-Propylenoxid-Addukte und insbesondere Alkylpolyalkylenglykolether.

Es ist bevorzugt, dass die Suspension neben dem Geliermittel nicht mehr als 2 Gew.-%, vorzugsweise nicht mehr als 1 Gew.-%, besonders bevorzugt nicht mehr als 0,55 Gew.-% organische Komponenten, enthält. Organische Komponenten schließen dabei organische Lösungsmittel und feste und flüssige organische Zusatzstoffe ein.

Es bestehen verschiedene Möglichkeiten, die Suspension des Zirkonoxid-Ausgangsmaterials, die das Geliermittel enthält, bereitzustellen. Das Zirkonoxid-Ausgangsmaterial kann z.B. mit dem Geliermittel gemischt und die erhaltene Mischung in dem flüssigen Medium suspendiert werden. Es ist auch möglich, das Geliermittel in festem oder flüssigem Zustand in die Suspension eines Zirkonoxid-Ausgangsmaterials zu geben.

In einer bevorzugten Ausführungsform wird zunächst das Geliermittel, z.B. Agarose, als Feststoff in die Suspension gegeben und dann die Suspension mit dem festen Geliermittel auf eine Temperatur erwärmt, bei der das Geliermittel in einen flüssigen Zustand übergeht.

In einer weiteren bevorzugten Ausführungsform wird das Geliermittel durch Erhitzen in einen flüssigen Zustand versetzt und dann in die Suspension gegeben, wobei die Suspension vorzugsweise eine Temperatur aufweist, bei der das Geliermittel in flüssigem Zustand bleibt.

Bei der Verwendung von Agarose als Geliermittel, kann z.B. Agarose zunächst in flüssiges Medium gegeben und auf eine Temperatur oberhalb des Schmelzpunkts von Agarose, z.B. auf über 88°C, erhitzt werden. Dieses Medium mit Agarose kann dann in die Suspension gegeben werden, wobei die Suspension eine Temperatur oberhalb der Temperatur aufweist, bei der die Agarose geliert, wie z.B. 70°C.

Das erfindungsgemäße Verfahren umfasst, dass mindestens zwei Suspensionen mit unterschiedlichen Zusammensetzungen geliert werden, wobei die Suspensionen oder die daraus hergestellten dichtgesinterten Körper unterschiedliche Farben, insbesondere unterschiedliche Farbwerte und/oder Helligkeiten, aufweisen.

Die unterschiedlichen Farben der Suspensionen werden üblicherweise dadurch erzielt, dass sich die Suspensionen hinsichtlich der Art und/oder der Menge an färbenden Elementen unterscheiden.

Die mindestens zwei Suspensionen mit unterschiedlicher Zusammensetzung werden direkt miteinander in Kontakt gebracht. Dazu wird zunächst eine erste Suspension mindestens teilweise geliert und dann die zweite Suspension auf die erste aufgetragen und ebenfalls geliert. Es ist bevorzugt, die erste Suspension mindestens 1 min, vorzugsweise mindestens 3 min und besonders bevorzugt mindestens 5 min, bei Umgebungsbedingungen abzukühlen, bevor die zweite Suspension auf die erste aufgetragen wird. In einer bevorzugten Ausführungsform wird die mindestens teilweise gelierte erste Suspension teilweise getrocknet, bevor die zweite Suspension aufgetragen wird.

Durch die Verwendung von Suspensionen mit unterschiedlichen Farben ist es möglich, mehrfarbige dentale Formkörper herzustellen, die die komplexe Farbgebung natürlicher Zähne imitieren. Es können zum Beispiel Suspensionen mit unterschiedlichen Farben übereinander gegeben werden, um Farbverläufe zu erzeugen.

Es können durch das erfindungsgemäße Verfahren auch komplexe Farbgebungen, wie z.B. dreidimensionale Farbverläufe, freie Schichtanordnungen, in denen die Schichten nicht ebenmäßig sind, und Kern-Schale-Anordnungen, erzeugt werden. In der Regel werden diese komplexen Farbgebungen erzeugt, indem eine erste Suspension mit Geliermittel in der gewünschten Gestalt geliert wird, bevor eine weitere Suspension aufgetragen und geliert wird. Die erste Suspension kann unter Verwendung einer Form in die gewünschte Gestalt gebracht werden und/oder kann vor dem Auftragen der weiteren Suspension nach Bedarf geformt werden.

In einer bevorzugten Ausführungsform werden zwei unterschiedlich gefärbte Suspensionen übereinander geschichtet, so dass sie eine schräg verlaufende Grenzfläche bilden. Die Grenzfläche kann im Wesentlichen geradlinig oder bogenförmig verlaufen. Bevorzugte Verläufe der schrägen Grenzfläche im Formkörper sind in der WO 2020/025795 A1 beschrieben. Beispielsweise ist es bevorzugt, dass die Grenzfläche in einer parallel zur Einschubachse verlaufenden Schnittebene durch den Formkörper einen Winkel von 10 bis 70°, vorzugsweise 10 bis 60°, zur Rotationsachse des Formkörpers und/oder eine Mamelonstruktur aufweist.

Es ist überraschenderweise festgestellt worden, dass durch das erfindungsgemäße Verfahren dentale Formkörper aus unterschiedlichen Suspensionen hergestellt werden können, wobei die Suspensionen im Formkörper einen festen Verbund bilden und der Verbund höchsten mechanischen Anforderungen genügt. Es konnte beispielsweise die hohe Gefügequalität an Grenzflächen gezeigt und ferner nachgewiesen werden, dass nach dem erfindungsgemäßen Verfahren hergestellte Formkörper in Materialtests nicht entlang der Grenzflächen brechen.

In einer Ausführungsform weist die in dem erfindungsgemäßen Verfahren verwendete Suspension vor dem Gelieren eine Viskosität von bis zu 25 Pas, insbesondere bis zu 10 Pas, besonders bevorzugt 0,001 bis 10 Pas auf. Die Viskosität wird, wenn nicht anders angegeben, mit einem Rotationsviskosimeter mit Platten-Kegel-System, Durchmesser 50 mm und Winkel 1° (MCR302-Modular Compact Rheometer, Firma Anton Paar GmbH) bei einer Scherrate im Bereich von 0,1 bis 5000 s⁻¹ und einer Temperatur unter 100°C gemessen.

Es wurde festgestellt, dass die Viskosität vor dem Gelieren in der Regel vom Anteil der festen Komponente in der Suspension, insbesondere von der eingesetzten Menge an Zirkonoxid-Ausgangsmaterial, sowie von der eingesetzten Menge an Geliermittel abhängig ist. Es wurde ferner festgestellt, dass ein höherer Gehalt an Zirkonoxid-Ausgangsmaterial und/oder Geliermittel in der Regel zu einer höheren Viskosität vor dem Gelieren führt.

In einer bevorzugten Ausführungsform des Verfahrens werden die Suspensionen in eine Form gegeben. Es ist bevorzugt, dass das Gelieren der Suspensionen mindestens teilweise in der Form erfolgt. Die Suspensionen können z.B. in die Form gegeben werden, indem sie gegossen oder gespritzt werden. Die Suspensionen können auch durch einen oder mehrere Gießkanäle in die Form gegeben werden. Die unterschiedlichen Suspensionen können gleichzeitig oder nacheinander in die Form gegeben werden.

Die Form entspricht vorzugsweise einer Negativform des dentalen Formkörpers, der durch das erfindungsgemäße Verfahren hergestellt werden soll. Es ist besonders bevorzugt, dass die Form einer vergrößerten Negativform des dentalen Formkörpers entspricht, wobei der Grad der Vergrößerung an die bei der weiteren Verarbeitung auftretende Schwindung angepasst ist.

Es hat sich gezeigt, dass im erfindungsgemäßen Verfahren auch der zeitliche Verlauf des Gelierens der Suspensionen, d.h. der Gelierprozess, in der Regel durch die Wahl geeigneter Verfahrensparameter kontrolliert werden kann. Geeignete Mittel zur Kontrolle des Gelierprozesses sind dabei insbesondere die eingesetzte Menge des Geliermittels, der Feststoffgehalt der Suspension und der gewählte Gelierparameter. In der Regel kann der Gelierprozess durch eine große Menge an Geliermittel, einen hohen Gehalt an Zirkonoxid-Ausgangsmaterial und/oder einen starken Einfluss des Gelierparameters, wie z.B. eine starke oder schnelle Temperaturänderung, gefördert werden.

Bei der Herstellung von dentalen Formkörpern aus mehreren Suspensionen kann es vorteilhaft sein, den Gelierprozess oder die Viskosität einer Suspension vor dem Gelieren gezielt an die zu erzielenden Formen anzupassen. Die gezielte Wahl der Fließeigenschaften der Suspensionen vor dem Gelieren und die Kontrolle des Gelierprozesses können hilfreich sein, um gewünschte Anordnungen der Suspensionen, insbesondere komplizierte dreidimensionale Anordnungen, zu erzielen.

Eine gelierte Suspension, die noch keiner Sinterung unterzogen wurde, wird im Sinne der Erfindung auch als Grünkörper bezeichnet.

In einer bevorzugten Ausführungsform des Verfahrens ist der hergestellte dentale Formkörper ein Grünkörper.

Der Grünkörper zeichnet sich vorzugsweise durch eine Dichte von 2,5 bis 4,0 g/cm³ und insbesondere 2,6 bis 3,8 g/cm³ und besonders bevorzugt 2,8 bis 3,6 g/cm³, aus.

Die Festigkeit der gelierten Suspension ist in der Regel von der Menge des in der Suspension enthaltenden Geliermittels und dem Gehalt an Zirkonoxid-Ausgangsmaterial abhängig. Insbesondere kann die Festigkeit in der Regel erhöht werden, indem mehr Geliermittel und/oder ein höherer Gehalt an Zirkonoxid-Ausgangsmaterial verwendet werden.

In einer Ausführungsform des Verfahrens werden die gelierten Suspensionen vor einer Trocknung in zwei oder mehrere Grünkörper geteilt, aus denen dann zwei oder mehrere dentale Formkörper hergestellt werden können. In einer weiteren Ausführungsform werden die gelierte Suspensionen einer Formgebung unterzogen. Die physikalischen Eigenschaften der gelierten Suspensionen, die noch keinem Trocknungsschritt unterzogen wurden, erlauben eine problemlose Teilung oder Bearbeitung durch maschinelle und insbesondere manuelle Verfahren.

Es hat sich gezeigt, dass es für das erfindungsgemäße Verfahren vorteilhaft sein kann, die gelierte Suspensionen auf eine Temperatur unterhalb der Raumtemperatur abzukühlen. In der Regel geht die Abkühlung mit einer Erhöhung der Viskosität/Festigkeit einher, was dazu führen kann, dass sich die gelierte Suspensionen besser bearbeiten oder aus einer Form entfernen lässt. Bei Verwendung eines Geliermittels, das durch Abkühlen geliert, kann in der Regel auch der Gelierprozess dadurch gefördert werden, dass die zu gelierende Suspensionen einer gekühlten Umgebung ausgesetzt wird. Es kann z.B. vorteilhaft sein, die zu gelierende Suspensionen in eine vorgekühlte Form zu geben, um eine schnelle Gelierung zu erzielen.

In einer bevorzugten Ausführungsform werden die Suspensionen auf eine Temperatur von weniger als 15°C, vorzugsweise weniger als 10°C, besonders bevorzugt 1°C bis 8°C, abgekühlt.

In einer bevorzugten Ausführungsform werden die gelierte Suspensionen, d.h. der Grünkörper, einer Trocknung unterzogen, um einen getrockneten Grünkörper herzustellen. In der Regel ist die Trocknung des Grünkörpers eine unvollständige Trocknung, d.h. der Gehalt des flüssigen Mediums im Grünkörper wird verringert, wobei auch nach der Trocknung noch flüssiges Medium im Grünkörper enthalten ist. Mit fortschreitender Trocknung verliert der Grünkörper typischerweise seine elastischen Eigenschaften, so dass ein getrockneter Grünkörper üblicherweise nicht mehr viskoelastisch ist. Wenn das Verfahren die Verwendung einer Form umfasst, kann die Trocknung der gelierten Suspensionen innerhalb der Form und/oder nach der Entnahme aus der Form erfolgen.

Es ist bevorzugt, dass die Trocknung für eine Dauer von mindestens 12 h, insbesondere mindestens 24 h, erfolgt.

Die Trocknung kann bei Umgebungsbedingungen oder unter kontrollierten Bedingungen, d.h. bei kontrollierter Temperatur und/oder Luftfeuchtigkeit, erfolgen. Vorzugsweise erfolgt die Trocknung bei einer Temperatur von 1°C bis 99°C, insbesondere 10°C bis 80°C, besonders bevorzugt 20°C bis 50°C und einer relativen Luftfeuchtigkeit von 0 bis 99%, insbesondere 1 bis 80% und besonders bevorzugt 1 bis 50%.

Die Trocknung des Grünkörpers kann beispielsweise für 24 h bei Umgebungsbedingungen durchgeführt werden.

Der getrocknete Grünkörper zeichnet sich vorzugsweise durch eine Dichte von 2,5 bis 4,0 g/cm³, insbesondere 2,6 bis 3,8 g/cm³ und besonders bevorzugt 2,8 bis 3,6 g/cm³, aus. Ferner weist der getrocknete Grünkörper vorzugsweise einen Feuchtigkeitsgehalt von bis zu 15 Gew.-%, insbesondere bis zu 10 Gew.-% und besonders bevorzugt bis zu 5 Gew.-%, auf.

In der Regel geht die Trocknung des Grünkörpers mit einer Schwindung einher. Vorzugsweise tritt bei der Trocknung des Grünkörpers eine lineare Schwindung von nicht mehr als 20%, insbesondere 10% bis 20%, auf. Die lineare Schwindung beim Trocknen ist die entlang einer Achse bestimmte prozentuale Längenreduktion, die beim Trocknen des Grünkörpers auftritt.

In einer bevorzugten Ausführungsform des erfindungsgemäßen Verfahrens wird der Grünkörper gesintert, insbesondere vorgesintert und/oder dichtgesintert.

Das Vorsintern erfolgt vorzugsweise bei einer Temperatur von 800°C bis 1300°C, insbesondere 850°C bis 1200°C, ganz besonders bevorzugt für 900°C bis 1150°C für insbesondere 1 bis 4 h, vorzugsweise 1,5 bis 2,5 h.

Das Dichtsintern erfolgt vorzugsweise bei einer Temperatur von 1200°C bis 1600°C, insbesondere 1300°C bis 1550°C und besonders bevorzugt bei 1350°C bis 1500°C für insbesondere 5 min bis 2 h, bevorzugt für 10 bis 60 min und besonders bevorzugt für 10 bis 30 min.

In einer bevorzugten Ausführungsform erfolgt während des Sinterns das Entbindern des Grünkörpers. Unter Entbindern wird das Ausbrennen der organischen Bestandteile, insbesondere der Geliermittel und Bindemittel verstanden. In einer weiteren Ausführungsform erfolgt das Entbindern in einem gesonderten Verfahrensschritt.

In der Regel geht nicht nur das Trocknen, sondern auch das Sintern mit einer Schwindung des Grünkörpers einher. Es ist bevorzugt, dass die lineare Schwindung der gelierten Suspension beim Trocknen und Sintern nicht mehr als 40%, insbesondere 20 bis 40%, besonders bevorzugt 30 bis 38%, beträgt. Die lineare Schwindung kann mithilfe eines Netzsch-Dilatometer DIL402 Supreme in einem Temperaturbereich von 20°C bis 1550°C bei einer Aufheizgeschwindigkeit von 2 K/min an Prüfkörpern ermittelt werden.

Da das Verfahren die Verwendung von unterschiedlichen Suspensionen umfasst, ist es vorteilhaft, die Zusammensetzungen der Suspensionen bezüglich der Schwindung, insbesondere der beim Sintern auftretenden Schwindung, aufeinander abzustimmen.

Es ist bevorzugt, dass der im erfindungsgemäßen Verfahren hergestellte dentale Formkörper ein dentaler Rohling oder eine dentale Restauration ist und vorzugsweise auf teilstabilisiertem tetragonalen, vollstabilisiertem kubischen Zirkonoxid oder deren Mischungen basiert. Typischerweise enthält die dentale Restauration im Wesentlichen kein monoklines Zirkonoxid.

Die Herstellung eines dentalen Rohlings ist besonders bevorzugt und umfasst in einer Ausführungsform, dass die gelierten Suspensionen vorgesintert werden. Vorzugsweise werden die gelierten Suspensionen getrocknet und dann vorgesintert.

In einer bevorzugten Ausführungsform weist der dentale Rohling die Form eines rechteckigen Blocks oder eines Zylinders auf.

In der Regel ist der hergestellte dentale Rohling dafür vorgesehen, zu einer dentalen Restauration verarbeitet zu werden. Dabei wird dem Rohling typischerweise mittels maschineller Bearbeitung, insbesondere unter Verwendung eines CAD/CAM-Verfahrens, die Form einer dentalen Restauration gegeben. Der Rohling kann an einen Halter gefügt werden, mit dem der Rohling in eine dafür vorgesehene Halterung einer CAD/CAM-Maschine einsetzt werden kann.

In einer bevorzugten Ausführungsform ist der Rohling einstückig mit dem Halter, besonders bevorzugt sind der Rohling und der Halter aus der gleichen oder aus unterschiedlichen Suspensionen hergestellt.

Typischerweise wird der im erfindungsgemäßen Verfahren hergestellte dentale Rohling zunächst einer Formgebung unterzogen und danach dichtgesintert, wodurch eine dentale Restauration mit den gewünschten Eigenschaften entsteht.

In einer weiteren besonders bevorzugten Ausführungsform ist der im erfindungsgemäßen Verfahren hergestellte dentale Formkörper eine dentale Restauration. Vorzugsweise ist die dentale Restauration eine Brücke, ein Inlay, ein Onlay, ein Veneer, ein Abutment, eine Teilkrone, eine Krone oder eine Schale.

Die dentale Restauration kann z.B. aus einem dentalen Rohling hergestellt werden, wobei die Formgebung durch maschinelle Bearbeitung, vorzugsweise in einem CAD/CAM-Verfahren erfolgt.

In einer weiteren bevorzugten Ausführungsform des erfindungsgemäßen Verfahrens umfasst die Herstellung der dentalen Restauration, dass die Suspension in eine Form gegeben wird, die einer Negativform einer dentalen Restauration entspricht, und die gelierte Suspension dichtgesintert wird. Vorzugsweise wird die gelierte Suspension getrocknet und dann dichtgesintert. Auf diese Weise kann die Formgebung mittels maschineller Bearbeitung vermieden oder der Aufwand einer maschinellen Bearbeitung reduziert werden.

Es ist bevorzugt, dass eine nach dieser Ausführungsform hergestellte dentale Restauration eine minimale Wandstärke von 0,12 mm oder mehr aufweist.

Diese Ausführungsform eignet sich auch besonders zur Herstellung von mehrfarbigen dentalen Restaurationen mit gegebenenfalls komplexen dreidimensionalen Farbverläufen. Dazu werden typischerweise zwei oder mehr Suspensionen mit unterschiedlichen Farben gleichzeitig oder nacheinander in die Form, die der Negativform der herzustellenden dentalen Restauration entspricht, gegeben.

Weiter entspricht bei dieser Ausführungsform die Form bevorzugt einer vergrößerten Negativform des dentalen Formkörpers, wobei der Grad der Vergrößerung an die im Verfahren auftretende Schwindung angepasst ist. Eine besonders genaue Anpassung der Vergrößerung an die Schwindung ermöglichen typischerweise solche Verfahren, bei denen die Form unter Verwendung eines digitalen Modells hergestellt wird.

Bei der Verwendung einer Form, die einer Negativform der dentalen Restauration entspricht, ist es bevorzugt, dass mindestens ein Teil der Form in einem additiven Verfahren oder durch maschinelle Bearbeitung, insbesondere in einem CAD/CAM-Verfahren, hergestellt ist. Diese Verfahren ermöglichen eine besonders genaue Gestaltung der Form unter Berücksichtigung der auftretenden Schwindung. In der Regel wird dabei die Form aus einem Material hergestellt, das nicht nur eine ausreichende Festigkeit bietet, sondern auch inert gegenüber der Geliermittel enthaltenden Suspension ist.

In einer weiteren bevorzugten Ausführungsform des Verfahrens, das die Verwendung einer Negativform der dentalen Restauration umfasst, wird die gelierte Suspension vorgesintert und gegebenenfalls einer maschinellen Feinbearbeitung unterzogen. Die gelierte Suspension, d.h. der Grünkörper, wird vorzugsweise erst getrocknet und dann vorgesintert.

Der vorgesinterte Grünkörper kann bei Bedarf auch einer maschinellen Bearbeitung unterzogen werden, beispielsweise um Materialrückstände zu entfernen, die durch Gießkanäle verursacht sind.

Es ist bevorzugt, dass ein vorgesinterter Grünkörper nach einer maschinellen Bearbeitung und/oder einer Feinbearbeitung dichtgesintert wird.

Die Erfindung betrifft ferner einen vorgesinterten dentalen Formkörper, der gemäß dem vorstehend beschriebenen erfindungsgemäßen Verfahren erhältlich ist. Dieser dentale Formkörper ist mehrfarbig und weist mindestens zwei unterschiedliche Farbwerte und/oder Helligkeiten auf.

Die nach dem erfindungsgemäßen Verfahren hergestellten dentalen Formkörper zeichnen sich typischerweise dadurch aus, dass sie eine komplexe mehrfarbige Farbgebung aufweisen können, die mit herkömmlichen Verfahren nicht erzeugt werden kann.

Die im erfindungsgemäßen Verfahren hergestellten dentalen Formkörper weisen vorteilhafte mechanische Eigenschaften auf.

In einer bevorzugten Ausführungsform weist der vorgesinterte dentale Formkörper, wie z.B. der vorgesinterte dentale Rohling, vorteilhafte Eigenschaften für eine maschinelle Bearbeitung auf. Es ist bevorzugt, dass dieser dentale Formkörper eine für ein CAD/CAM-Verfahren vorteilhafte Vickers-Härte von 300 bis 1000 MPa aufweist. Die Vickers-Härte kann bei einer Kraft im Bereich von 2,5 bis 5,0 kbf (24,517 bis 49,034 N) und insbesondere bei einer Kraft von 5,0 kgf (49,034 N) gemäß ISO 14705:2008 gemessen werden.

Ferner liegt der vorgesinterte Formkörper typischerweise in einem offenporigen Zustand vor, wobei der vorgesinterte Formkörper vorzugsweise eine Dichte von 30%, insbesondere 40 bis 75% der Dichte der dichtgesinterten Zirkonoxid-Keramik hat.

Ein nach dem erfindungsgemäßen Verfahren hergestellter dichtgesinterter dentaler Formkörper, wie eine dichtgesinterte dentale Restauration, weist vorzugsweise eine Biaxialfestigkeit von mindestens 500 MPa, insbesondere mindestens 600 MPa und besonders bevorzugt mindestens 800 MPa, auf.

Ferner weist der dichtgesinterte dentale Formkörper vorzugsweise Dichte von mehr als 5,8 g/cm³, insbesondere mehr als 5,9 g/cm³ und besonders bevorzugt mehr als 6,0 g/cm³ auf.

Die Erfindung betrifft auch die Verwendung einer Suspension eines Zirkonoxid-Ausgangsmaterials als Dentalmaterial, insbesondere zur Herstellung eines dentalen Formkörpers, wobei die Suspension ein Geliermittel enthält und geliert wird.

Alle Verfahrensschritte, Verfahrensparameter und stofflichen Definitionen, die im Rahmen des erfindungsgemäßen Verfahrens beschrieben wurden, sind auch für diese erfindungsgemäße Verwendung geeignet.

Die Erfindung wird im Folgenden anhand von Ausführungsbeispielen näher erläutert.

### Beispiele

### Beispiel 1

Es wurde nach dem erfindungsgemäßen Verfahren ein mehrfarbiger Prüfkörper aus zwei unterschiedlichen Suspensionen hergestellt.

Dazu wurden zunächst zwei Suspensionen von Zirkonoxid-Ausgangsmaterial hergestellt. Die erste Suspension enthielt 72 Gew.-% eines mit 3 Mol-% Y₂O₃ stabilisierten Zirkonoxid-Pulvers (Z-Pex, Tosoh Corporation) sowie 27,68 Gew.-% Wasser als flüssiges Medium, 0,25 Gew.-% Dispergiermittel (Dolapix^{®} CE64, Zschimmer & Schwarz), 0,06 Gew.-% Entschäumer (Contraspum^{®} K1012, Zschimmer & Schwarz) und 0,01 Gew.-% NH₄OH zur Einstellung des pH-Werts, jeweils bezogen auf das Gesamtgewicht der Suspension, und sie wies eine weiße Färbung auf.

Die zweite Suspension enthielt 72 Gew.-% eines mit 5 Mol-% stabilisierten Zirkonoxid-Pulvers (Z-Pex smile, Tosoh Corporation) sowie 28 Gew.-% Wasser als flüssiges Medium, und sie wies eine gelbe Färbung auf.

Beide Suspensionen wurden auf 70°C erhitzt. Es wurde eine wässrige Agarose-Lösung hergestellt, auf 90°C erhitzt und zu den erhitzten Suspensionen gegeben, so dass die Suspensionen 0,2 Gew.-% Agarose, bezogen auf die erhitzte Suspension, enthielten.

Die erhitzten und mit Agarose versetzten Suspensionen wurden dann mit einem zeitlichen Abstand von mehr als 1 min nacheinander in eine Form gegeben.

Mit dem Gießen der Suspensionen in die Form begann das Abkühlen der Suspensionen. Das Abkühlen wurde zusätzlich dadurch unterstützt, dass die Form mit den Suspensionen für 1 Stunde bei 8°C gelagert wurde.

Die abgekühlten und bei der Abkühlung gelierten Suspensionen, die auch als Grünkörper bezeichnet werden, wurden entformt.

Der Grünkörper wurde für 24 h bei Umgebungsbedingungen getrocknet und anschließend gesintert. Dafür wurde, wie für alle in den Beispielen genannten Sinterschritte, ein Ofen des Typs Programat der Ivoclar Vivadent AG verwendet.

Der Grünkörper wurde z.B. bei 950°C für 2 h vorgesintert.

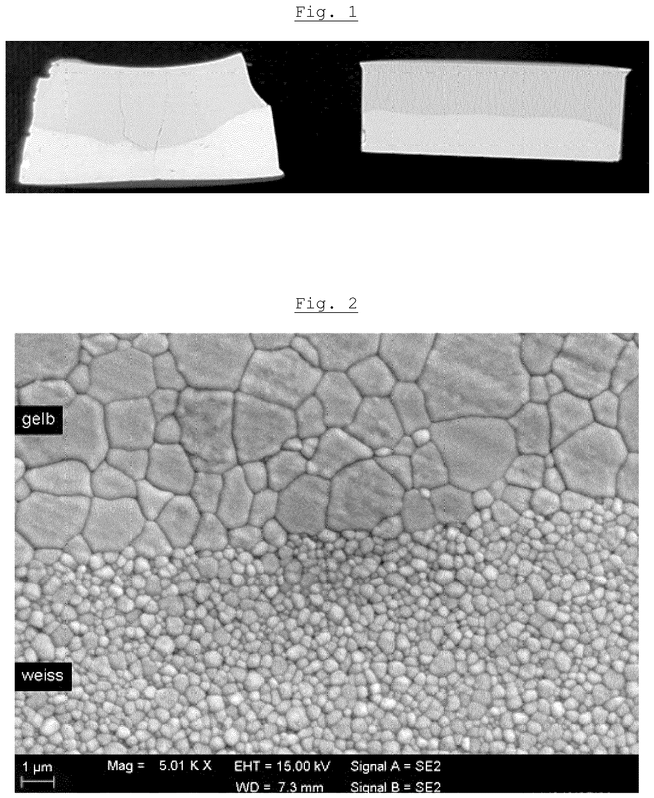

Die Figur 1 zeigt Querschnitte durch vorgesinterte Prüfkörper. Durch unterschiedliche Gießverfahren, wie z.B. durch eine Variation der Gießgeschwindigkeit, konnten unterschiedliche Schichtanordnungen erzeugt werden. Es konnten beispielsweise im Wesentlichen ebenmäßige (rechts) und freie (links) Schichtanordnungen erzeugt werden. Die freien Schichtanordnungen wiesen z.B. schiefe Ebenen oder wellenförmige Schichten auf.

Es wurden auch Grünkörper nach dem vorstehenden Verfahren hergestellt, getrocknet und bei 1500°C für 25 min dichtgesintert.

Figur 2 zeigt eine rasterelektronenmikroskopische Aufnahme der Grenzfläche zwischen den zwei unterschiedlichen Suspensionen nach dem Dichtsintern. Es zeigt sich, dass das Gefüge an der Grenzfläche zwischen der gelben (oben) und der weißen (unten) Schicht keine Poren aufweist. Auch innerhalb der Schichten aus dichtgesinterten Zirkonoxid-Materialien zeigten sich keine Poren.

Die Figuren 1 und 2 veranschaulichen, dass Suspensionen von unterschiedlichen Zirkonoxid-Ausgangsmaterialien, die Geliermittel enthalten, übereinander gegeben werden können, ohne dass sich die Suspensionen miteinander vermischen.

### Beispiel 2

Es wurden mehrfarbige Prüfkörper zur Bestimmung der Biaxialfestigkeit nach dem erfindungsgemäßen Verfahren hergestellt.

Es wurden zwei Suspensionen mit jeweils 66,5 Gew.-% Zirkonoxid-Ausgangsmaterial hergestellt, wobei für die Herstellung der ersten Suspension weißes Z-Pex smile (Tosoh Corporation) und für die zweite Suspension gelbes Z-Pex smile yellow (Tosoh Corporation) verwendet wurde und die Suspensionen Dolapix CE64, Contraspum K1012 und NH₄OH in den für Beispiel 1 angegebenen Mengen enthielten. Zu den Suspensionen wurden nach dem für Beispiel 1 beschriebenen Verfahren jeweils 0,2 Gew.-% Agarose, bezogen auf das Gesamtgewicht der Suspension, gegeben.

Dann wurde zunächst die erste mit Agarose versetzte Suspension in eine liegende zylindrische Kunststoffform (Durchmesser ca. 25 mm; Länge ca. 30 mm) gegeben, so dass die Form teilweise gefüllt war. Die erste Suspension wurde geliert, indem die Kunststoffform mit der ersten Suspension für etwa 20 min auf 8°C abgekühlt wurde. Danach wurde die zweite mit Agarose versetzte Suspension in die Form gefüllt und geliert, indem die Kunststoffform für 1 h auf 8°C abgekühlt wurde.

Danach wurde der gelierte zylindrische Grünkörper entformt und für 24 h bei Umgebungsbedingungen getrocknet. Der getrocknete Grünkörper wurde für 2 h bei 950°C vorgesintert und dann in etwa 2 mm dicken Scheiben geteilt. Diese wurden daraufhin bei 1500°C für 25 min dichtgesintert und dann einer Bruchfestigkeitsprüfung nach ISO 6872 unterzogen.

Die dichtgesinterten Prüfkörper wiesen eine Biaxialfestigkeit von 418 bis 654 MPa auf, bestimmt nach ISO 6872.

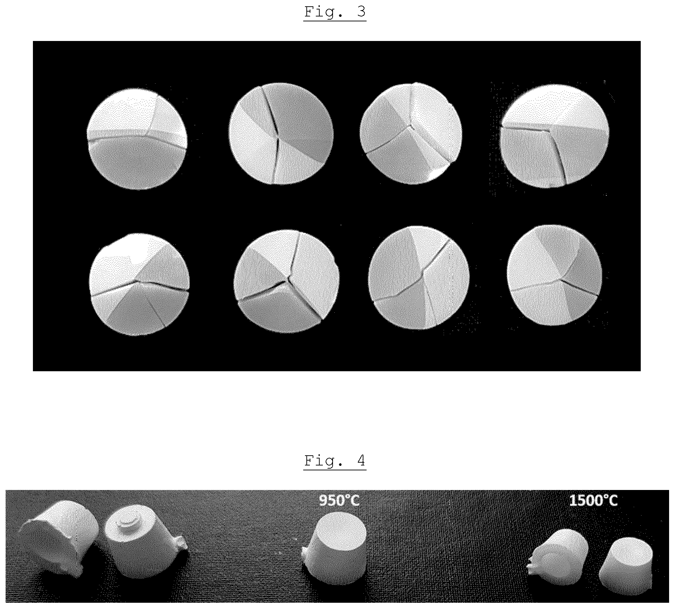

Die Figur 3 zeigt die Prüfkörper nach Bestimmung der Biaxialfestigkeit. Es zeigt sich, dass ein Bruch zufällig durch das Material und nicht entlang der Grenzflächen der unterschiedlichen Schichten erfolgte. Dies veranschaulicht, dass eine sehr hohe Verbundstärke zwischen den Schichten erzielt werden kann.

### Beispiele 3 bis 6

Es wurden die Geliereigenschaften von Suspensionen mit den in den Tabellen 1 und 2 angegebenen Zusammensetzungen untersucht. Dazu wurden Suspensionen mit unterschiedlichen Mengen eines mit 5 Mol-% Y₂O₃ stabilisierten Zirkonoxid-Pulvers (Z-Pex smile, Tosoh Corporation) in Wasser hergestellt. Zu diesen Suspensionen wurden nach dem für Beispiel 1 beschriebenen Verfahren unterschiedliche Mengen Agarose gegeben. Für die Herstellung von Beispiel 3 wurde von diesem Verfahren abgewichen und die pulverförmige Agarose wurde direkt mit in die Suspension gegeben, ohne vorher eine Agarose-Lösung herzustellen. Die Agarose enthaltenden Suspensionen wurden in konische Zylinderformen gegeben und für 1 Stunde bei 8°C abgekühlt. Die Viskosität der Suspensionen vor der Gelierung und Beobachtungen zur Gelierung sind in den Tabellen 1 und 2 angegeben.

Die Grünkörper wurden aus der Form entnommen, 24 h bei Umgebungsbedingungen getrocknet, bei 950°C für 2 h vorgesintert und dann bei 1500°C für 25 min dichtgesintert.

**Tabelle 1**

| **Beispiel** | **3** | **4** |
|---|---|---|
| Zirkonoxid-Ausgangsmaterial (Gew.-%) | 66, 5 | 69,8 |
| Agarose (Gew.-%, bezogen auf H₂O) | 0, 6 | 0,3 |
| Agarose (Gew.-%,) bezogen auf Gesamtgewicht | 0,201 | 0,091 |
| Viskosität vor Gelierung | leicht viskos | viskos |
| Gelierung | Fester Grünkörper nach 20 min bei 8°C | Fester Grünkörper nach 20 min bei 8°C |
| Dichte nach Dichtsintern (g/cm³) | 6, 01 | 6, 011 |

**Tabelle 2**

| **Beispiel** | **5** | **6** |
|---|---|---|
| Zirkonoxid-Ausgangsmaterial (Gew.-%) | 72, 6 | 69, 8 |
| Agarose (Gew.-%, bezogen auf H₂O) | 0,1 | 0,5 |
| Agarose (Gew.-%,) bezogen auf Gesamtgewicht | 0, 027 | 0, 15 |
| Viskosität vor Gelierung | hoch viskos | sehr hoch viskos |
| Gelierung | Fester Grünkörper, Gelierung unmittelbar nach Agarose-Zugabe | Fester Grünkörper nach 2 min, Gelierung unmittelbar nach Agarose-Zugabe |

Die Dichte der dichtgesinterten Grünkörper wurde nach der Archimedes-Methode bestimmt.

Die mechanischen Eigenschaften des Formkörpers von Beispiel 3 wurden untersucht. Nach dem Trocknen wies der Formkörper eine Dichte von 2,71 g/cm³ auf und seine Biaxialfestigkeit war zu gering, um sie nach ISO 6872 zu bestimmen. Die Restfeuchte des Formkörpers nach dem Trocknen wurde unter Verwendung eines Halogen-Feuchtemessgeräts (HR83, Mettler Toledo) durch eine Stufentrocknung bis 200°C bestimmt und sie betrug 3,9 Gew.-%. Nach dem Vorsintern wies der Formkörper eine Dichte von 2,77 g/cm³ auf und die Biaxalfestigkeit nach ISO 6872 betrug 13 MPa. Nach dem Dichtsintern wies der Formkörper eine Biaxialfestigkeit von 595 MPa auf.

Die Versuche zeigen, dass durch einen hohen Gehalt an Zirkonoxid-Ausgangsmaterial oder eine große Menge Agarose in der Suspension die Viskosität der Suspension vor der Gelierung erhöht werden kann.

Ferner zeigen die Beispiele, dass bei einem höheren Gehalt an Zirkonoxid-Ausgangsmaterial eine geringere Menge an Agarose ausreichen kann, um durch die Gelierung einen festen Grünkörper zu erzeugen.

Die Beispiele zeigen auch, dass durch eine größere Menge Agarose eine höhere Festigkeit des Grünkörpers bewirkt werden kann.

### Beispiele 7 und 8

Für die Beispiele 7 und 8 wurden Suspensionen entsprechend den Beispielen 3 und 4 hergestellt. Ferner wurden mittels Stereolitographie Formen hergestellt, die der Negativform eines Fingerhuts entsprachen. Nach Zugabe der Agarose wurden die Suspensionen in die Formen gegossen und für 1 Stunde bei 8°C gekühlt. Die gelierten Suspensionen wurden entformt und für 24 h bei Umgebungsbedingungen getrocknet.

Die getrockneten Suspensionen, d.h. die getrockneten Grünkörper, wurden bei 950°C für 2 h vorgesintert oder bei 1500°C für 25 min dichtgesintert. Die Dichte der dichtgesinterten Körper betrug 6,03 g/cm³.

Die Figur 4 zeigt getrocknete Grünkörper (links), vorgesinterte (Mitte) und dichtgesinterte (rechts) Körper des Beispiels 7. Die dargestellten Körper veranschaulichen, dass auch Körper mit komplizierten Formen und dünnen Wandstärken erhalten werden können.

## Patentansprüche

1. Verfahren zur Herstellung eines dentalen Formkörpers, bei dem mindestens zwei Suspensionen eines Zirkonoxid-Ausgangsmaterials mit unterschiedlichen Zusammensetzungen geliert werden, indem eine erste Suspension mindestens teilweise geliert wird und dann eine zweite Suspension auf die erste aufgetragen und ebenfalls geliert wird, wobei die Suspensionen ein Geliermittel enthalten und wobei die Suspensionen unterschiedliche Farben aufweisen.

2. Verfahren nach Anspruch 1, bei dem die Suspensionen 55 bis 70 Gew.-%, vorzugsweise 60 bis 70 Gew.-%, besonders bevorzugt 65 bis 70 Gew.-% Zirkonoxid-Ausgangsmaterial enthalten.

3. Verfahren nach einem der Ansprüche 1 bis 2, bei dem das Zirkonoxid-Ausgangsmaterial Y₂O₃, La₂O₃, CeO₂, MgO und/oder CaO, vorzugsweise in einer Menge von 2 bis 14 Mol-%, insbesondere 2 bis 8 Mol-%, besonders bevorzugt 3 bis 5 Mol-%, bezogen auf den Gehalt an Zirkonoxid, enthält.

4. Verfahren nach einem der Ansprüche 1 bis 3, bei dem die Suspensionen durch Abkühlen geliert werden.

5. Verfahren nach einem der Ansprüche 1 bis 4, bei dem das Geliermittel ein Polysaccharid, ein Protein oder eine Mischung davon, vorzugsweise Agarose, Agar, Alginat, Gellan, Carrageen, Pektin, Chitosan, Chitin, Gelatine oder eine Mischung davon, und besonders bevorzugt Agarose, ist.

6. Verfahren nach einem der Ansprüche 1 bis 5, bei dem die Suspensionen 0,03 bis 0,5 Gew.-% und vorzugsweise 0,03 bis 0,4 Gew.-% Agarose enthalten.

7. Verfahren nach einem der Ansprüche 1 bis 6, bei dem die Suspensionen neben dem Geliermittel nicht mehr als 2 Gew.-%, vorzugsweise nicht mehr als 1 Gew.-%, besonders bevorzugt nicht mehr als 0,55 Gew.-% organische Komponenten enthalten.

8. Verfahren nach einem der Ansprüche 1 bis 7, bei dem die Suspensionen in eine Form gegeben werden.

9. Verfahren nach einem der Ansprüche 1 bis 8, bei dem die gelierten Suspensionen viskoelastisch und vorzugsweise entformbar sind.

10. Verfahren nach einem der Ansprüche 1 bis 9, bei dem die Suspensionen auf eine Temperatur von weniger als 15°C, vorzugsweise weniger als 10°C, besonders bevorzugt 1°C bis 8°C, abgekühlt werden.

11. Verfahren nach einem der Ansprüche 1 bis 10, bei dem der dentale Formkörper ein dentaler Rohling oder eine dentale Restauration ist und vorzugsweise auf teilstabilisiertem tetragonalen, vollstabilisiertem kubischen Zirkonoxid oder deren Mischungen basiert.

12. Verfahren nach Anspruch 11, bei dem die dentale Restauration eine Brücke, ein Inlay, ein Onlay, ein Veneer, ein Abutment, eine Teilkrone, eine Krone oder eine Schale ist.

13. Verfahren nach Anspruch 11 oder 12, bei dem die Herstellung der dentalen Restauration umfasst, dass die Suspensionen in eine Form gegeben werden, die einer Negativform einer dentalen Restauration entspricht, und die gelierten Suspensionen dichtgesintert werden.

14. Verfahren nach Anspruch 13, bei dem mindestens ein Teil der Form in einem additiven Verfahren oder durch maschinelle Bearbeitung, insbesondere in einem CAD/CAM-Verfahren, hergestellt ist.

15. Verfahren nach Anspruch 13 oder 14, bei dem die gelierten Suspensionen vorgesintert und gegebenenfalls einer maschinellen Bearbeitung unterzogen werden.

16. Verfahren nach Anspruch 11, bei dem die Herstellung des dentalen Rohlings umfasst, dass die gelierten Suspensionen vorgesintert werden.

17. Vorgesinterter dentaler Formkörper, der gemäß dem Verfahren nach einem der Ansprüche 1 bis 16 erhältlich ist.

18. Verwendung einer Suspension eines Zirkonoxid-Ausgangsmaterials als Dentalmaterial, insbesondere zur Herstellung eines dentalen Formkörpers, vorzugsweise eines dentalen Rohlings oder einer dentalen Restauration, wobei mindestens zwei Suspensionen eines Zirkonoxid-Ausgangsmaterials mit unterschiedlichen Zusammensetzungen geliert werden, indem eine erste Suspension mindestens teilweise geliert wird und dann eine zweite Suspension auf die erste aufgetragen und ebenfalls geliert wird, wobei die Suspensionen ein Geliermittel enthalten und wobei die Suspensionen unterschiedliche Farben aufweisen.

## Claims

1. Process for the preparation of a dental shaped body, in which at least two suspensions of a zirconium oxide starting material with different compositions are gelled by at least partially gelling a first suspension and then applying a second suspension to the first and gelling it as well, wherein the suspensions comprise a gelling agent and wherein the suspensions have different colors.

2. Process according to claim 1, in which the suspensions comprise 55 to 70 wt.-%, preferably 60 to 70 wt.-%, particularly preferably 65 to 70 wt.-% of zirconium oxide starting material.

3. Process according to any one of claims 1 to 2, in which the zirconium oxide starting material comprises Y₂O₃, La₂O₃, CeO₂, MgO and/or CaO, preferably in an amount of 2 to 14 mol%, in particular 2 to 8 mol%, particularly preferably 3 to 5 mol%, based on the amount of zirconium oxide.

4. Process according to any one of claims 1 to 3, in which the suspensions are gelled by cooling.

5. Process according to any one of claims 1 to 4, in which the gelling agent is a polysaccharide, a protein or a mixture thereof, preferably agarose, agar, alginate, gellan, carrageenan, pectin, chitosan, chitin, gelatin or a mixture thereof, and particularly preferably agarose.

6. Process according to any one of claims 1 to 5, in which the suspensions comprise 0.03 to 0.5 wt.-% and preferably 0.03 to 0.4 wt.-% of agarose.

7. Process according to any one of claims 1 to 6, in which the suspensions comprise, in addition to the gelling agent, not more than 2 wt.-%, preferably not more than 1 wt.-%, particularly preferably not more than 0.55 wt.-% of organic components.

8. Process according to any one of claims 1 to 7, in which the suspensions are placed in a mould.

9. Process according to any one of claims 1 to 8, in which the gelled suspensions are viscoelastic and preferably demouldable.

10. Process according to any of claims 1 to 9, in which the suspensions are cooled to a temperature of less than 15°C, preferably less than 10°C, particularly preferably 1°C to 8°C.

11. Process according to any one of claims 1 to 10, in which the dental shaped body is a dental blank or a dental restoration and is preferably based on partially stabilized tetragonal, fully stabilized cubic zirconium oxide or mixtures thereof.

12. Process according to claim 11, in which the dental restoration is a bridge, inlay, onlay, veneer, abutment, partial crown, crown or facet.

13. Process according to claim 11 or 12, in which the preparation of the dental restoration comprises placing the suspensions in a mould corresponding to a negative shape of a dental restoration and densely sintering the gelled suspensions.

14. Process according to claim 13, in which at least a part of the mould is produced in an additive process or by machining, in particular in a CAD/CAM process.

15. Process according to claim 13 or 14, in which the gelled suspensions are presintered and, optionally, subjected to machining.

16. Process according to claim 11, in which the preparation of the dental blank comprises that the gelled suspensions are presintered.

17. Presintered dental shaped body obtainable by the process according to any one of claims 1 to 16.

18. Use of a suspension of a zirconium oxide starting material as dental material, in particular for the preparation of a dental shaped body, preferably a dental blank or a dental restoration, wherein at least two suspensions of a zirconium oxide starting material with different compositions are gelled by at least partially gelling a first suspension and then applying a second suspension to the first and gelling it as well, wherein the suspensions comprise a gelling agent and wherein the suspensions have different colors.

## Revendications

1. Procédé de fabrication d'un corps moulé dentaire, selon lequel au moins deux suspensions d'un matériau de départ en zircone sont gélifiées avec des compositions différentes, en gélifiant au moins partiellement une première suspension, et en appliquant ensuite une deuxième suspension sur la première et en la gélifiant à son tour, les suspensions contenant un gélifiant, et les suspensions présentant des couleurs différentes.

2. Procédé selon la revendication 1, selon lequel les suspensions contiennent de 55 à 70 % en poids, de préférence de 60 à 70 % en poids, de manière particulièrement avantageuse de 65 à 70 % en poids de matériau de départ en zircone.

3. Procédé selon l'une des revendications 1 à 2, selon lequel le matériau de départ en zircone contient Y₂O₃, La₂O₃, CeO₂, MgO et/ou CaO, de préférence en une quantité allant de 2 à 14 % en moles, notamment de 2 à 8 % en moles, de manière particulièrement avantageuse de 3 à 5 % en moles, rapportée à la teneur en zircone.

4. Procédé selon l'une des revendications 1 à 3, selon lequel les suspensions sont gélifiées par refroidissement.

5. Procédé selon l'une des revendications 1 à 4, selon lequel l'agent gélifiant est un polysaccharide, une protéine ou un mélange de ceux-ci, de préférence de l'agarose, de l'agar-agar, un alginate, la gel-lane, le carraghénane, la pectine, un chitosan, la chitine, la gélatine ou un mélange de ceux-ci, et de manière particulièrement avantageuse de l'agarose.

6. Procédé selon l'une des revendications 1 à 5, selon lequel les suspensions contiennent de 0,03 à 0,5 % en poids et de préférence de 0,03 à 0,5 % en poids d'agarose.

7. Procédé selon l'une des revendications 1 à 6, selon lequel les suspensions contiennent, outre le gélifiant, pas plus de 2 % en poids, de préférence pas plus de 1 % en poids, de manière particulièrement avantageuse pas plus de 0,55 % en poids de composants organiques.

8. Procédé selon l'une des revendications 1 à 7, selon lequel les suspensions sont placées dans un moule.

9. Procédé selon l'une des revendications 1 à 8, selon lequel les suspensions gélifiées sont viscoélastiques et, de préférence, démoulables.

10. Procédé selon l'une des revendications 1 à 9, selon lequel les suspensions sont refroidies jusqu'à une température inférieure à 15 °C, de préférence inférieure à 10 °C, de manière particulièrement avantageuse allant de 1 °C à 8 °C.

11. Procédé selon l'une des revendications 1 à 10, selon lequel le corps moulé dentaire est une ébauche dentaire ou une restauration dentaire et est de préférence basé sur de la zircone tétragonale partiellement stabilisée, de la zircone cubique totalement stabilisée ou des mélanges de celles-ci.

12. Procédé selon la revendication 11, selon lequel la restauration dentaire est un bridge, un inlay, un onlay, une facette dentaire, un pilier, une couronne partielle, une couronne ou une coque.

13. Procédé selon la revendication 11 ou 12, selon lequel la fabrication de la restauration dentaire comprend le fait que les suspensions sont placées dans un moule qui correspond à une forme négative d'une restauration dentaire, et les suspensions gélifiées sont frittées à densité maximale.

14. Procédé selon la revendication 13, selon lequel au moins une partie du moule est fabriquée avec un procédé additif ou par usinage mécanique, notamment avec un procédé CAO/FAO.

15. Procédé selon la revendication 13 ou 14, selon lequel les suspensions gélifiées sont soumises à un préfrittage et, le cas échéant, à un usinage mécanique.

16. Procédé selon la revendication 11, selon lequel la fabrication de l'ébauche dentaire comprend le fait que les suspensions gélifiées sont préfrittées.

17. Corps moulé dentaire préfritté, qui peut être obtenu selon le procédé selon l'une des revendications 1 à 16.

18. Utilisation d'une suspension d'un matériau de départ en zircone en tant que matériau dentaire, notamment pour la fabrication d'un corps moulé dentaire, de préférence d'une ébauche dentaire ou d'une restauration dentaire, où au moins deux suspensions d'un matériau de départ en zircone sont gélifiées avec des compositions différentes, par le fait qu'une première suspension est gélifiée au moins partiellement, et ensuite une deuxième suspension est appliquée sur la première et est également gélifiée, les suspensions contenant un agent gélifiant, et les suspensions présentant des couleurs différentes.
